(19) **Europäisches Patentamt / European Patent Office / Office européen des brevets**

(11) **EP 4 715 073 A1**

(12) # EUROPEAN PATENT APPLICATION
published in accordance with Art. 153(4) EPC

(43) Date of publication:
**25.03.2026 Bulletin 2026/13**

(21) Application number: **23937520.7**

(22) Date of filing: **17.05.2023**

(51) International Patent Classification (IPC):
*C22C 14/00* (2006.01)     *C22F 1/00* (2006.01)
*C22F 1/18* (2006.01)     *A61K 6/84* (2020.01)
*A61L 27/06* (2006.01)     *A61B 5/00* (2006.01)

(52) Cooperative Patent Classification (CPC):
**A61B 5/00; A61K 6/84; A61L 27/06; C22C 14/00;
C22F 1/00; C22F 1/18**

(86) International application number:
**PCT/JP2023/018481**

(87) International publication number:
**WO 2024/236782 (21.11.2024 Gazette 2024/47)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Sumitomo Electric Industries, Ltd.
Osaka-shi, Osaka 541-0041 (JP)**

(72) Inventors:
• **TANGE, Yoshinori
Osaka-shi, Osaka 541-0041 (JP)**
• **NISHIYAMA, Norimasa
Osaka-shi, Osaka 541-0041 (JP)**

(74) Representative: **Boult Wade Tennant LLP
Salisbury Square House
8 Salisbury Square
London EC4Y 8AP (GB)**

(54) **TITANIUM MATERIAL, MEDICAL MEMBER, DENTAL IMPLANT CONSTITUTING MEMBER, AND CAPSULE FOR HOUSING DIAMOND SENSOR**

(57) A titanium material includes 91% by mass or more of titanium, wherein an electrical resistivity ω μΩ·m of the titanium material and a content ratio c % by mass of a component of the titanium material other than the titanium indicates a relation of the following formula I: ω>0.16c+0.56 Formula I, and in the formula I, c is 0 or more and 9 or less.

FIG.4

EP 4 715 073 A1

**Description**

TECHNICAL FIELD

The present disclosure relates to a titanium material, a member for medical use, a dental implant component member, and a capsule for accommodating a diamond sensor.

BACKGROUND ART

Titanium materials, since having a high specific strength, have been used in the fields of aerospace industries, automotive industries and the like. Further, titanium materials, since being excellent in biocompatibility, have been in high demand as metal materials for living bodies, such as dental implants.

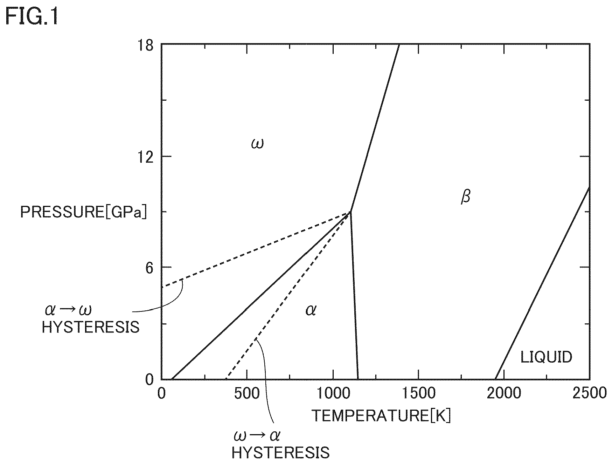

As shown in a temperature-pressure phase diagram of titanium of Fig. 1, in the titanium, there are three phases of alpha titanium having a crystal structure of an alpha phase (phase indicated as $\alpha$ in Fig. 1), beta titanium having a crystal structure of a beta phase (phase indicated as $\beta$ in Fig. 1), and omega titanium having a crystal structure of an omega phase (phase indicated as $\omega$ in Fig. 1). The alpha titanium is a stable phase at room temperature and ambient pressure, and has a hexagonal close-packed (hcp) crystal structure. The beta titanium is a stable phase on the higher temperature side, and has a body-centered cubic (bcc) crystal structure. The omega titanium is a metastable transition phase formed when the alpha titanium is crystallized from the beta titanium, and has a simple hexagonal crystal structure.

Patent Literature 1 discloses, as a titanium material having a high strength, a titanium material having $\alpha$ phase and $\omega$ phase present in a mixed state at room temperature and ambient pressure.

CITATION LIST

PATENT LITERATURE

PTL 1: Japanese Patent Laying-Open No. 2009-228053

SUMMARY OF INVENTION

A titanium material of the present disclosure is a titanium material comprising 91% by mass or more of titanium, wherein

an electrical resistivity $\omega$ $\mu\Omega\cdot$m of the titanium material and a content ratio c % by mass of a component of the titanium material other than the titanium indicates a relation of the following formula I:

$$\omega > 0.16c + 0.56 \qquad \text{Formula I,}$$

and
in the formula I, c is 0 or more and 9 or less.

BRIEF DESCRIPTION OF DRAWINGS

[Fig. 1] Fig. 1 is a temperature-pressure phase diagram of titanium.
[Fig. 2] Fig. 2 shows a coordinate system indicating a relation between tensile strength $\sigma B$ and fracture elongation $\delta$ of each of conventional titanium materials and a titanium material of Embodiment 1.
[Fig. 3] Fig. 3 is a schematic cross section of a high-pressure cell of an ultrahigh-pressure and high-temperature apparatus used to manufacture the titanium material of the present disclosure.
[Fig. 4] Fig. 4 shows a coordinate system indicating a relation between electrical resistivity $\omega$ of a titanium material of each sample of examples and a content ratio c of a component of the titanium material other than titanium.

DETAILED DESCRIPTION

[Problem to be Solved by the Present Disclosure]

An implant consisting of a titanium material is implanted into a bone, an osteoblast cell is conglutinated to a surface of the implant, and is grown to couple the implant and the bone together. As the surface of the implant is rougher, the osteoblast cell is more likely to be conglutinated. One of techniques for roughening the surface of the implant consisting of the titanium material is anodization treatment. In the anodization treatment, the implant is immersed in an electrolyte solution and is electrolyzed with the implant serving as an anode, thereby attaining a thick titanium oxide ($TiO_2$) film at the surface of the implant.

By controlling the shape of the surface of the titanium oxide film, adhesion between the titanium oxide film and the osteoblast cell is improved. In the anodization treatment, as the electrical resistivity of the material used as the anode is larger, the shape of the surface tends to be more readily controlled. Therefore, a titanium material having a high electrical resistivity has been required.

Thus, it is an object of the present disclosure to provide a titanium material having a high electrical resistivity, as well as a member for medical use, a dental implant component member, and a capsule for accommodating a diamond sensor, each of which includes the titanium material.

[Advantageous Effect of the Present Disclosure]

According to the present disclosure, it is possible to provide a titanium material having a high electrical resistivity, as well as a member for medical use, a dental implant component member, and a capsule for accommodating a diamond sensor, each of which includes the titanium material.

[Description of Embodiments]

First, embodiments of the present disclosure will be listed and described.

(1) A titanium material of the present disclosure is a titanium material comprising 91% by mass or more of titanium, wherein

an electrical resistivity $\omega$ $\mu\Omega\cdot$m of the titanium material and a content ratio c % by mass of a component of the titanium material other than the titanium indicates a relation of the following formula I:

$$\omega > 0.16c + 0.56 \qquad \text{Formula I,}$$

and
in the formula I, c is 0 or more and 9 or less.

The titanium material of the present disclosure can have a high electrical resistivity. In the present disclosure, the description "the titanium material has a high electrical resistivity" means that the electrical resistivity of the titanium material of the present disclosure is higher than the electrical resistivity of a conventional titanium material which has the same content ratio of titanium as that of the titanium material of the present disclosure and in which the titanium is alpha titanium.

(2) In (1), the titanium material may include 49% by mass or more of titanium having a crystal structure of an omega phase. According to this, the titanium material can have excellent strength and ductility.

(3) In (1) or (2), the titanium material may include 98.8% by mass or more of the titanium. According to this, biocompatibility of the titanium material is improved.

(4) In any one of (1) to (3), a tensile strength $\sigma$B MPa of the titanium material and a fracture elongation $\delta$ % of the titanium material may indicate a relation of the following formula II:

$$\sigma B \geq 1600 - 30\delta \qquad \text{Formula II,}$$

and
in the formula II, $\sigma$B$\geq$400 and $\delta\geq$20.
According to this, the titanium material can have high strength and high ductility.

(5) In any one of (1) to (4), an average grain diameter of crystal grains constituting the titanium material may be 1 $\mu$m or more and 1000 $\mu$m or less. According to this, the titanium material can have high strength and high ductility.

(6) In any one of (1) to (5), a Vickers hardness of the titanium material may be 200 Hv or more. According to this, the titanium material can have a high hardness.

(7) In any one of (1) to (6), a heat-resistant temperature of the titanium material may be 100°C or more. According to this, the titanium material can maintain a high strength even at a high temperature of 100°C or more.

(8) In any one of (1) to (7), a volume of the titanium material may be 0.001 mm$^3$ or more. According to this, since the titanium material has a sufficient size as a metal material for living bodies, the titanium material can be used for various purposes of use such as a dental implant component member and an artificial joint. Further, the titanium material can also be suitably used as a material of a capsule for accommodating a diamond sensor.

(9) In any one of (1) to (8), the titanium material may include 98.8% by mass or more of the titanium,

the titanium material may include at least one impurity element selected from a group consisting of hydrogen, carbon, nitrogen, oxygen, and iron, and

a total content ratio of the titanium and the impurity element in the titanium material may be 99.99% by mass or more. According to this, the titanium material can have excellent biocompatibility.

(10) In any one of (1) to (9), a proportion D90/D10 of a cumulative 90% grain diameter D90 from a small diameter side to a cumulative 10% grain diameter D10 from the small diameter side in a cumulative grain size distribution based on volume of crystal grains constituting the titanium material may be 5 or more and 1000 or less. According to this, the strength and the ductility of the titanium material are homogenized and the titanium material can have a high strength and a high ductility.

(11) A member for medical use according to the present disclosure is a member for medical use, the member comprising the titanium material according to any one of (1) to (10). The member for medical use according to the present disclosure can have a high electrical resistivity.

(12) A dental implant component member according to the present disclosure is a dental implant component member comprising the titanium material according to any one of (1) to (10). The dental implant component member according to the present disclosure can have a high electrical resistivity.

(13) A capsule for accommodating a diamond sensor according to the present disclosure is a capsule for accommodating a diamond sensor, the capsule comprising the titanium material according to any one of (1) to (10). The capsule for accommodating a diamond sensor according to the present disclosure can have a high electrical resistivity.

[Detailed Description of Embodiments]

Specific examples of the titanium material, the member for medical use, the dental implant component member, and the capsule for accommodating a diamond sensor according to the present disclosure will be described below with reference to drawings. In the drawings of the present disclosure, the same reference sign represents the same part or a corresponding part. Then, the dimensional relation among such as length, width, thickness, and depth is suitably varied for clarification and simplification of the drawings, and does not always represent an actual dimensional relation.

In the present disclosure, the notation in the form of "A to B" means the upper limit and the lower limit in a range (that is, A or more and B or less), and in the case where A has no description of a unit and only B has a description of a unit, the unit of A and the unit of B are the same.

In the present disclosure, when one or more numerical values are described as each of the lower and upper limits of a numerical range, it is assumed that a combination of any one numerical value described as the lower limit and any one numerical value described as the upper limit is also disclosed. For example, in the case where a1 or more, b1 or more, and c1 or more are each described as the lower limit and a2 or less, b2 or less, and c2 or less are each described as the upper limit, it is assumed that a1 or more and a2 or less, a1 or more and b2 or less, a1 or more and c2 or less, b1 or more and a2 or less, b1 or more and b2 or less, b1 or more and c2 or less, c1 or more and a2 or less, c1 or more and b2 or less, and c1 or more and c2 or less are disclosed.

[Embodiment 1: Titanium Material]

A titanium material according to one embodiment of the present disclosure (hereinafter, also referred to as "Embodiment 1") is a titanium material comprising 91% by mass or more of titanium, wherein

an electrical resistivity $\omega$ ($\mu\Omega\cdot$m) of the titanium material and a content ratio c % by mass of a component of the titanium material other than the titanium indicates a relation of the following formula I:

$$\omega > 0.16c + 0.56 \qquad \text{Formula I,}$$

and
in the formula I, c is 0 or more and 9 or less.

The titanium material of Embodiment 1 has a high electrical resistivity. In the formula I, the slope is 0.16. This indicates that the electrical resistivity of the titanium material tends to be more likely to be increased as the content ratio c of the component of the titanium material other than the titanium is increased.

The electrical resistivity $\omega$ ($\mu\Omega\cdot$m) of the titanium material and the content ratio c (% by mass) of the component of the titanium material other than the titanium can indicate a relation of the following formula I-A or the following formula I-B:

$$\omega > 0.16c + 0.62 \qquad \text{Formula I-A,}$$

$$\omega > 0.16c + 0.68 \qquad \text{Formula I-B,}$$

and in each of the formulas I-A and I-B, c is 0 or more and 9 or less.

The titanium material satisfying the formula I-A or the formula I-B may have a further higher electrical resistivity.

<Composition>

The titanium material according to Embodiment 1 includes 91% by mass or more of the titanium. The lower limit of the content ratio of the titanium of the titanium material may be 93% by mass or more, 95% by mass or more, 98% by mass or more, 98.8% by mass or more, 98.80% by mass or more, 98.90% by mass or more, 98.955% by mass or more, 98.96% by mass or more, 99.0% by mass or more, 99.20% by mass or more, 99.205% by mass or more, 99.30% by mass or more, 99.325% by mass or more, 99.40% by mass or more, 99.495% by mass or more, 99.99% by mass or more, or 99.999% by mass or more from the viewpoint of improving biocompatibility. The upper limit of the content ratio of the titanium of the titanium material may be 100% by mass or less. The titanium material can also consist of 100% by mass of the titanium. The content ratio of the titanium of the titanium material may be 91% by mass or more and 100% by mass or less, 93% by mass or more and 100% by mass or less, 95% by mass or more and 100% by mass or less, 98% by mass or more and 100% by mass or less, 98.8% by mass or more and 100% by mass or less, 98.955% by mass or more and 100% by mass or less, 99.0% by mass or more and 100% by mass or less, 99.205% by mass or more and 100% by mass or less, 99.325% by mass or more and 100% by mass or less, 99.495% by mass or more and 100% by mass or less, or 99.999% by mass or more and 100% by mass or less.

The upper limit of the content ratio of the titanium of the titanium material according to Embodiment 1 may be, for example, 99.9999% by mass or less or 99.999% by mass or less when an inevitable impurity is taken into consideration. The content ratio of the titanium of the titanium material may be 91% by mass or more and 99.9999% by mass or less, 93% by mass or more and 99.9999% by mass or less, 95% by mass or more and 99.9999% by mass or less, 98% by mass or more and 99.9999% by mass or less, 98.8% by mass or more and 99.9999% by mass or less, 98.955% by mass or more and 99.9999% by mass or less, 99.0% by mass or more and 99.9999% by mass or less, 99.205% by mass or more and 99.9999% by mass or less, 99.325% by mass or more and 99.9999% by mass or less, 99.495% by mass or more and 99.9999% by mass or less, or 99.9990% by mass or more and 99.9999% by mass or less.

The content ratio c of the component of the titanium material according to Embodiment 1 other than the titanium is 0% by mass or more and 9% by mass or less. Examples of the component other than the titanium include: a general transition metal element (scandium (Sc), chromium (Cr), manganese (Mn), iron (Fe), cobalt (Co), nickel (Ni), copper (Cu), yttrium (Y), zirconium (Zr), niobium (Nb), molybdenum (Mo), silver (Ag), hafnium (Hf), tantalum (Ta), tungsten (W), platinum (Pt), gold (Au), or the like); and hydrogen (H), carbon (C), nitrogen (N), or oxygen (O) each serving as an inevitable impurity.

In the present disclosure, a method for measuring the content ratio c of the component of the titanium material other than the titanium is as follows. A total content ratio c1 (% by mass) of all the metal elements (Sc, Cr, Mn, Fe, Co, Ni, Cu, Y, Zr, Nb, Mo, Ag, Hf, Ta, W, Pt, Au, and the like) of the titanium material other than the titanium is measured by performing ICP spectroscopy (high-frequency inductively coupled plasma emission spectroscopy) onto the titanium material. By performing SIMS spectrometry (secondary ion mass spectrometry) onto the titanium material, a total content ratio c2 (% by mass) of all the elements (such as carbon (C), nitrogen (N), oxygen (O), and hydrogen (H)) other than the metal elements of the titanium material is measured. In the present disclosure, the total of the total content ratio c1 and the total content ratio c2 corresponds to the content ratio c of the component of the titanium material other than the titanium.

In the present disclosure, the content ratio of the titanium of the titanium material is determined by the following method: the content ratio c of the component other than the titanium is measured by the above-described method and the content ratio c of the component other than the titanium is subtracted from 100% by mass of the titanium material.

In Embodiment 1, the titanium material may include 98.8% by mass or more of the titanium, the titanium material may include at least one impurity element selected from a group consisting of hydrogen, carbon, nitrogen, oxygen and iron, and the total content ratio of the titanium and the impurity element in the titanium material may be 99.99% by mass or more. Thus, since the titanium material includes no component harmful to living bodies, such as vanadium (V) and aluminum (Al), which are included in the conventional titanium alloy, or even if such a component is included, includes a very small amount of the component, the titanium material can have excellent biocompatibility.

The total content ratio of the titanium and the impurity element in the titanium material may be 99.99% by mass or more and 100% by mass or less, 99.999% by mass or more and 100% by mass or less, or 100% by mass.

<Electrical Resistance ω>

The lower limit of the electrical resistivity ω of the titanium material of Embodiment 1 is more than 0.56 μΩ·m, may be 0.60 μΩ·m or more, may be 0.70 μΩ·m or more, may be 0.78 μΩ·m or more, may be 0.80 μΩ·m or more, may be 0.82 μΩ·m or more, may be 0.85 μΩ·m or more, may be 0.90 μΩ·m or more, may be 0.95 μΩ·m or more, may be 0.98 μΩ·m or more, may be 1.00 μΩ·m or more, or may be 1.50 μΩ·m or more. The upper limit of the electrical resistivity ω of the titanium material of Embodiment 1 is 2.00 μΩ·m or less. The electrical resistivity ω of the titanium material is more than 0.56 μΩ·m and 2.00 μΩ·m or less, may be 0.78 μΩ·m or more and 2.00 μΩ·m or less, may be 0.82 μΩ·m or more and 2.00 μΩ·m or less, may be 0.90 μΩ·m or more and 2.00 μΩ·m or less, or may be 1.00 μΩ·m or more and 2.00 μΩ·m or less.

In the present disclosure, the method for measuring the electrical resistivity of the titanium material is as follows. The shapes of measuring samples are each configured to have a cylindrical shape having a diameter of about 8 mm and a height of about 10 mm. The electrical conductivity (MS/m) of each of the measuring samples is measured using an eddy current type portable conductivity measurement device ("SIGMATEST" (trademark) from Foerster Japan). The measurement is performed onto the upper surface of the cylinder. The measurement is performed five times, and the average of the electrical conductivities obtained in the respective measurements (average of the five electrical conductivities) is calculated. The electrical resistivity (μΩ·m) is obtained from the inverse number of the average of the electrical conductivities (MS/m). It should be noted that it has been confirmed that the electrical conductivity is measured five times or more for the same measuring sample and there is substantially no variation in measurement result (for example, 5% or less).

<Titanium Having Crystal Structure of Omega Phase>

The titanium material of Embodiment 1 can include 49% by mass or more of titanium having a crystal structure of an omega phase. Thereby, the titanium material can have excellent strength and ductility.

Conventionally, the presence of the omega titanium at room temperature and ambient pressure has been confirmed as being precipitated as nano grains in a trace amount in the alpha titanium phase in the manufacture process of alpha pure titanium including about 99% by mass or more of alpha titanium. The omega titanium causes the alpha pure titanium to become brittle. Accordingly, it has conventionally been considered that it is preferable to reduce the content of the omega titanium in the alpha pure titanium.

As a result of try-and-error under the idea completely opposite to the conventional technical idea of reducing the content of the omega titanium in the alpha pure titanium, the present inventors have produced a titanium material including 49% by mass or more of the omega titanium. The titanium material including 49% by mass or more of the omega titanium has been confirmed to have excellent strength and ductility.

The lower limit of the content ratio (hereinafter, also referred to as "the content ratio of the omega titanium") of the titanium having the crystal structure of the omega phase in the titanium material may be 49% by mass or more, 50% by mass or more, 55% by mass or more, 60% by mass or more, 65% by mass or more, 70% by mass or more, 75% by mass or more, 80% by mass or more, 85% by mass or more, 90% by mass or more, 95% by mass or more, 98.8% by mass or more, 98.955% by mass or more, 99% by mass or more, 99.2% by mass or more, 99.205% by mass or more, 99.325% by mass or more, 99.495% by mass or more, 99.5% by mass or more, or 99.999% by mass or more from the viewpoint of improving strength and ductility. The upper limit of the content ratio of the omega titanium in the titanium material may be 100% by mass or less. The titanium material can also consist of 100% by mass of the omega titanium. The content ratio of the omega titanium in the titanium material may be 49% by mass or more and 100% by mass or less, 50% by mass or more and 100% by mass or less, 55% by mass or more and 100% by mass or less, 60% by mass or more and 100% by mass or less, 65% by mass or more and 100% by mass or less, 70% by mass or more and 100% by mass or less, 75% by mass or more and 100% by mass or less, 80% by mass or more and 100% by mass or less, 85% by mass or more and 100% by mass or less, 90% by mass or less and 100% by mass or less, 95% by mass or less and 100% by mass or less, 98.8% by mass or more and 100% by mass or less, 99% by mass or more and 100% by mass or less, 99.2% by mass or more and 100% by mass or less, or 99.999% by mass or more and 100% by mass or less.

The upper limit of the content ratio of the omega titanium in the titanium material of Embodiment 1 can be, for example, 99.9999% by mass or less when an inevitable impurity is taken into consideration. The content ratio of the omega titanium in the titanium material may be 49% by mass or more and 99.9999% by mass or less, 50% by mass or more and 99.9999% by mass or less, 55% by mass or more and 99.9999% by mass or less, 60% by mass or more and 99.9999% by mass or less, 65% by mass or more and 99.9999% by mass or less, 70% by mass or more and 99.9999% by mass or less, 75% by mass or more and 99.9999% by mass or less, 80% by mass or more and 99.9999% by mass or less, 85% by mass or more and 99.9999% by mass or less, 90% by mass or more and 99.9999% by mass or less, 95% by mass or more and 99.9999% by mass or less, 98.8% by mass or more and 99.9999% by mass or less, 99% by mass or more and 99.9999% by mass or less, 99.2% by mass or more and 99.9999% by mass or less, or 99.9990% by mass or more and 99.9999% by mass or less.

The titanium material of Embodiment 1 may include one or both of alpha titanium and beta titanium in addition to the omega titanium as long as the effects of the present disclosure are exhibited. The total content ratio of the alpha titanium and the beta titanium in the titanium material can be a value obtained by subtracting the content ratio of the omega titanium from the content ratio of the titanium of the titanium material.

In the titanium material of Embodiment 1, a percentage of the omega titanium to the total of the alpha titanium, the beta titanium and the omega titanium based on mass may be 50% or more and 100% or less, 55% or more and 100% or less, 60% or more and 100% or less, 65% or more and 100% or less, 70% or more and 100% or less, 75% or more and 100% or less, 80% or more and 100% or less, 85% or more and 100% or less, 90% or less and 100% or less, 95% or less and 100% or less, 98.8% or more and 100% or less, 99% or more and 100% or less, 99.2% or more and 100% or less, 99.999% or more and 100% or less, or 100%. The percentage of the omega titanium to the total of the alpha titanium, the beta titanium and the omega titanium based on mass corresponds to the percentage of the omega titanium of the titanium based on mass.

The content ratio (% by mass) of the omega titanium in the titanium material is measured by the following procedure. First, the content ratio c of the component of the titanium material other than the titanium is measured by the ICP spectroscopy and the SIMS spectrometry. The content ratio (% by mass) of the titanium of the titanium material is obtained by subtracting the content ratio c of the component of the titanium material other than the titanium from 100% by mass of the titanium material. Next, the titanium material is subjected to X-ray diffraction measurement to obtain an X-ray diffraction spectrum.

Examples of the device used for the X-ray diffraction measurement include "MiniFlex" (trademark) provided by Rigaku. X-ray diffraction measurement conditions are as follows.

<<X-Ray Diffraction Measurement Conditions>>

Characteristic X-ray: Cu-K$\alpha$ (wavelength of 1.54 Å)
Filter: multilayer mirror
Optical system: concentration method
X-Ray diffraction method: $\theta$-2$\theta$ method
Temperature at the time of measurement: 25°C

In the obtained X-ray diffraction spectrum, the intensities of the omega titanium, the alpha titanium and the beta titanium are measured. The intensity I$\omega$ of the omega titanium is the maximum intensity in a range of 2$\theta$=38.8° to 39.2°. The intensity I$\alpha$ of the alpha titanium is the maximum intensity in a range of 2$\theta$=39.4° to 40.6°. The intensity I$\beta$ of the beta titanium is the maximum intensity in a range of 2$\theta$=38.2. By calculating a proportion I$\omega$/(I$\omega$+I$\omega$+I$\beta$) of the intensity I$\omega$ to the total of the intensity I$\omega$, the intensity I$\alpha$, and the intensity I$\beta$, the percentage (%) of the omega titanium of the titanium based on mass is obtained. The content ratio (% by mass) of the omega titanium in the titanium material is calculated based on the content ratio (% by mass) of the titanium in the titanium material and the percentage (%) of the omega titanium in the titanium material based on mass.

<Relation between Tensile Strength $\sigma$B MPa and Fracture Elongation $\delta$ %>

In the titanium material of Embodiment 1, the tensile strength $\sigma$B MPa and the fracture elongation $\delta$ % of the titanium material can indicate a relation of the following formula II:

$$\sigma B \geq 1600\text{-}30\delta \qquad \text{Formula II, and}$$

and
in the formula II, $\sigma$B$\geq$400 and $\delta$$\geq$20.

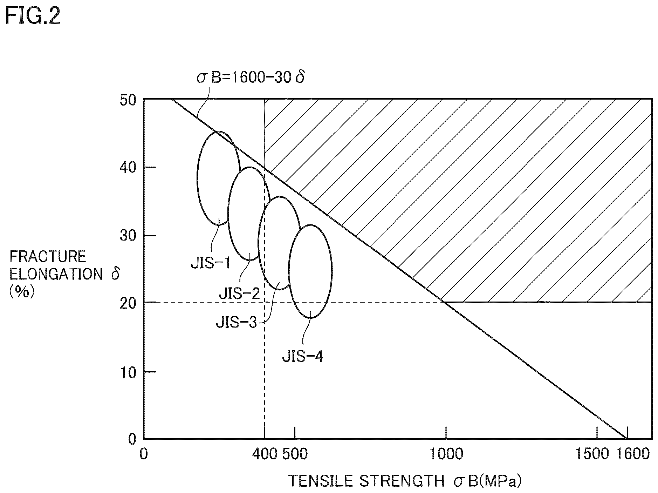

The titanium material having the relation of the formula II will be described with reference to Fig. 2. Fig. 2 shows a

# EP 4 715 073 A1

coordinate system indicating a relation between the tensile strength σB and the fracture elongation δ of the titanium material. In the coordinate system of Fig. 2, the X axis indicates the tensile strength σB (MPa), and the Y axis indicates the fracture elongation δ (%). The tensile strength is one index indicating the strength of a material, and the index indicates that the higher the numerical value, the higher the strength. The fracture elongation is one index indicating the ductility of a material, and the index indicates that the higher the numerical value, the higher the ductility. In Fig. 2, conventional titanium materials are shown as JIS-1 to JIS-4 and the data of the tensile strength and fracture elongation of each of the conventional titanium materials were prepared by making reference to Fig. 1 in Hideki Fujii, Takashi Maeda (2013), "Titanium Alloys Developed by Nippon Steel & Sumitomo Metal Corporation", Shinnittetsu Sumikin giho, No. 396, pp.16-22.

Each of the JIS-1 to JIS-4 means industrial pure titanium described in JIS H 4600:2012 "Titanium and titanium alloys - Sheets, plates and strips". Specifically, the JIS-1 means JIS H 4600 Class 1; the JIS-2 means JIS H 4600 Class 2; the JIS-3 means JIS H 4600 Class 3; and the JIS-4 means JIS H 4600 Class 4. Each of the JIS-1 to JIS-4 has a content ratio of titanium of about 99% by mass or more, and have a crystal structure of an alpha phase. Hereinafter, the pure titanium having the crystal structure of the alpha phase is referred to also as alpha pure titanium.

In Fig. 2, the region having the relation of the above formula II is a region indicated by oblique lines. The region indicated by the oblique lines is the region where the fracture elongation is 20% or more, high in ductility, and the tensile strength is 400 MPa or more, high in strength. The titanium material satisfying the relation of the above formula II has a high strength and a high ductility. The alpha pure titanium, which is the conventional titanium material, has a high fracture elongation (hereinafter, also referred to as ductility), but has a low tensile strength (also referred to as strength), and does not satisfy the relation of the formula II.

The tensile strength σB MPa and the fracture elongation δ % of the titanium material can indicate a relation of the following formula II-A or the following formula II-B:

$$\sigma B > 1875 - 30\delta \qquad \text{Formula II-A}$$

$$\sigma B > 1900 - 30\delta \qquad \text{Formula II-B,}$$

and

in each of the formulas II-A and II-B, σB≥400 and δ≥20.

The titanium material satisfying the relation of the formula II-A or the formula II-B can have higher strength and higher ductility.

<Tensile Strength αB>

The lower limit of the tensile strength σB of the titanium material of Embodiment 1 can be 400 MPa or more. The lower limit of the tensile strength σB of the titanium material may be 500 MPa or more, 600 MPa or more, or 800 MPa or more from the viewpoint of securing excellent strength. The upper limit of the tensile strength σB of the titanium material is not particularly limited, but can be, for example, less than 1550 MPa. The tensile strength σB of the titanium material may be 400 MPa or more and less than 1550 MPa, 500 MPa or more and less than 1550 MPa, 600 MPa or more and less than 1550 MPa, or 800 MPa or more and less than 1550 MPa.

The measurement of the tensile strength σB of the titanium material is carried out according to JIS Z 2241:2011 "Metal materials -- Tensile testing -- Method of test at room temperature". The test temperature is set at 23°C±5°C.

<Fracture Elongation δ>

The fracture elongation δ of the titanium material of Embodiment 1 can be 20% or more. The lower limit of the fracture elongation δ of the titanium material may be 25% or more, 30% or more, or 35% or more from the viewpoint of securing excellent ductility. The upper limit of the fracture elongation δ of the titanium material may be, for example, 50% or less, or 45% or less. The fracture elongation δ of the titanium material may be 20% or more and 50% or less, 25% or more and 50% or less, 30% or more and 50% or less, 35% or more and 50% or less, 20% or more and 45% or less, 25% or more and 45% or less, or 30% or more and 45% or less.

The measurement of the fracture elongation δ of the titanium material is carried out according to JIS Z 2241:2011 "Metal materials -- Tensile testing -- Method of test at room temperature". The test temperature is set at 23°C±5°C.

<Average Grain Diameter>

The average grain diameter of the crystal grains constituting the titanium material of Embodiment 1 (hereinafter, referred to also as "average grain diameter of the titanium material") can be 1 μm or more and 1000 μm or less. Thereby, the strength and the ductility of the titanium material are more improved.

The lower limit of the average grain diameter of the titanium material may be 1 μm or more, 3 μm or more, 5 μm or more, 10 μm or more, or 20 μm or more from the viewpoint of improving the strength. The upper limit of the average grain diameter of the titanium material may be 1000 μm or less, 500 μm or less, 200 μm or less, 100 μm or less, or 50 μm or less from the viewpoint of securing excellent strength. The average grain diameter of the titanium material may be 1 μm or more and 1000 μm or less, 3 μm or more and 500 μm or less, 5 μm or more and 200 μm or less, 10 μm or more and 100 μm or less, 10 μm or more and 50 μm or less, or 20 μm or more and 50 μm or less.

In the present disclosure, the average grain diameter of the titanium material is measured by an intercept procedure. A specific measuring procedure is as follows. A surface of the titanium material is polished with SiC abrasive paper and $Al_2O_3$ lapping film. The polished surface is imaged by using an optical microscope at a magnification of 100 times to obtain an optical microscopic image.

A circle of 50 mm in diameter is drawn on the optical microscopic image, 8 straight lines are radially drawn from the center of the circle to the outer periphery thereof, and the number of times each of the straight lines crosses grain boundaries in the circle is counted. The length of the straight line is divided by the number of the times the straight line crosses the grain boundaries so as to determine an average intercept length, and the average intercept length is multiplied by a conversion factor, 1.128, to a two-dimensional grain diameter, thereby obtaining a value as the average grain diameter.

The above measurement is carried out at three positions for one measuring sample, and the average value of the average grain diameters at the three positions is taken as the average grain diameter of the titanium material in the present disclosure.

It should be noted that as far as the applicant has performed the measurement, it has been confirmed that as long as the measurement is performed onto the same sample, even when the measurement of the average grain diameter of the titanium material is performed a plurality of times with the measuring positions being changed, there is substantially no variation in measurement result.

It is preferable, from the viewpoint of homogenizing strength and ductility, that the grain diameters of the crystal grains constituting the titanium material are low in variation. The proportion D90/D10 of a cumulative 90% grain diameter D90 from a small diameter side to a cumulative 10% grain diameter D10 from the small diameter side in a cumulative grain size distribution based on volume of the crystal grains constituting the titanium may be 5 or more and 1000 or less, or may be 10 or more and 1000 or less. It is indicated that the less the value of D90/D10, the less the variation in the grain diameters of the crystal grains.

The grain diameter of each crystal grain for calculating the D90/D10 is determined by carrying out image processing using a commercially available image analysis software on an optical microscopic image taken under the same condition as in the above intercept procedure, to measure an equivalent circle diameter of each crystal grain. A measuring visual field of 50 mm × 50 mm is set in the optical microscopic image, and a volume-base cumulative grain size distribution is formed based on all crystal grains observed in the measuring visual field. The D90/D10 is calculated based on the cumulative grain size distribution.

<Vickers Hardness>

The Vickers hardness of the titanium material of Embodiment 1 can be 200 Hv or more. According to this, the titanium material has excellent hardness and has improved wear resistance.

The lower limit of the Vickers hardness of the titanium material may be 200 Hv or more or 220 Hv or more from the viewpoint of securing excellent hardness. Since a higher upper limit of the Vickers hardness of the titanium material is more preferable, the upper limit can be, for example, 400 Hv or less although the upper limit is not particularly limited. The Vickers hardness of the titanium material may be 200 Hv or more and 400 Hv or less, or may be 220 Hv or more and 400 Hv or less.

The measurement of the Vickers hardness of the titanium material is carried out according to JIS Z 2244:2009 "Vickers hardness test - Test method". The test temperature is set at 23°C±5°C.

It should be noted that as far as the applicant has performed the measurement, it has been confirmed that as long as the measurement is performed onto the same sample, even when the Vickers hardness of the titanium material is measured a plurality of times with the measurement positions being changed, there is substantially no variation in measurement result.

<Heat-Resistant Temperature>

The heat-resistant temperature of the titanium material of Embodiment 1 can be 100°C or more. Thereby, the titanium material can maintain excellent strength even at a high temperature of 100°C or more.

The lower limit of the heat-resistant temperature of the titanium material of Embodiment 1 may be 100°C or more, 120°C or more, or 140°C or more from the viewpoint of securing excellent strength. Since a higher upper limit of the heat-resistant temperature of the titanium material is more preferable, the upper limit can be, for example, 190°C or less although the upper limit is not particularly limited. The heat-resistant temperature of the titanium material may be 100°C or more and 190°C or less, 120°C or more and 190°C or less, or 140°C or more and 190°C or less.

The heat-resistant temperature of the titanium material is measured by comparing an X-ray diffraction pattern at 25°C with X-ray diffraction patterns at predetermined temperatures through X-ray diffractometry. A specific measuring method for the measurement is as follows.

A measuring sample is prepared by polishing the surface of the titanium material. The measuring sample is irradiated with X rays under the following conditions by using an X-ray diffraction device to obtain X-ray diffraction patterns. A plurality of temperatures of 25°C and exceeding 25°C in the measurement are suitably selected and the X-ray diffraction patterns are obtained at the respective temperatures.

<<X-Ray Diffraction Measurement Conditions>>

Characteristic X-ray: Cu-K$\alpha$ (wavelength of 1.54 Å)
Filter: multilayer mirror
Optical system: concentration method
X-ray diffraction method: $\theta$-$2\theta$ method

The X-ray diffraction pattern at 25°C and each of the X-ray diffraction patterns at the predetermined temperatures exceeding 25°C (hereinafter, referred to also as "predetermined temperatures") are compared; and in the case where the shapes of both of the X-ray diffraction patterns coincide, it is determined that the crystal structure of the measuring sample is maintained at the predetermined temperature, and the measuring sample has heat resistance. Here, "both of the X-ray diffraction patterns coincide" is confirmed by coincidences of all the diffraction peak positions, and also coincidences of the orders of the intensities of the respective diffraction peaks.

The above X-ray diffraction measurement is carried out by raising the temperature condition until the X-ray diffraction pattern at the predetermined temperature exceeding 25°C has a shape different from that of the ray diffraction pattern at 25°C. Among the plurality of X-ray diffraction patterns obtained, an X-ray diffraction pattern at a highest temperature coinciding with the X-ray diffraction pattern at 25°C is specified. The highest temperature is taken as the heat-resistant temperature of the measuring sample.

<Volume>

The volume of the titanium material of Embodiment 1 can be 0.001 mm$^3$ or more. Since the titanium material has a sufficient size as a metal material for living bodies, the titanium material can be used for various purposes of use such as a dental implant component member and an artificial joint. Further, the titanium material can also be suitably used as a material of a capsule for accommodating a diamond sensor.

The lower limit of the volume of the titanium material may be 0.001 mm$^3$ or more, 0.01 mm$^3$ or more, 0.1 mm$^3$ or more, 1 mm$^3$ or more, 10 mm$^3$ or more, or 100 mm$^3$ or more. A higher upper limit of the volume of the titanium material is more preferable, and the upper limit is preferably, for example, 100000 mm$^3$ or less although the upper limit is not particularly limited. The volume of the titanium material may be 0.001 mm$^3$ or more and 100000 mm$^3$ or less, 10 mm$^3$ or more and 100000 mm$^3$ or less, or 100 mm$^3$ or more and 100000 mm$^3$ or less. The volume of the titanium material is measured by Archimedes' method.

<0.2% Yield Strength in Tensile Test>

The 0.2% yield strength of the titanium material of Embodiment 1 in a tensile test can be more than 180 MPa. Thus, the strength is further improved.

The lower limit of the 0.2% yield strength of the titanium material in the tensile test may be 250 MPa or more, 400 MPa or more, or 550 MPa or more from the viewpoint of securing excellent strength. A higher upper limit of the 0.2% yield strength of the titanium material in the tensile test is more preferable, and the upper limit is therefore not particularly limited.

The measurement of the 0.2% yield strength of the titanium material in the tensile test is carried out according to JIS Z 2241:2011 "Metal materials -- Tensile testing -- Method of test at room temperature". The test temperature is set at 23°C±5°C.

<0.2% Yield Strength in Compression Test>

The 0.2% yield strength of the titanium material of Embodiment 1 in a compression test can be 570 MPa or more. Thus, the strength is further improved.

The lower limit of the 0.2% yield strength of the titanium material in the compression test may be 600 MPa or more, 700 MPa or more, or 800 MPa or more from the viewpoint of securing excellent strength. Since a higher upper limit of the 0.2% yield strength of the titanium material in the compression test is more preferable, the upper limit can be, for example, 5000 MPa or less although the upper limit is not particularly limited. The 0.2% yield strength of the titanium material in the compression test may be 570 MPa or more and 5000 MPa or less, may be 600 MPa or more and 5000 MPa or less, may be 700 MPa or more and 5000 MPa or less, or may be 800 MPa or more and 5000 MPa or less.

The measurement of the 0.2% yield strength of the titanium material in the compression test is carried out according to JIS R 1608:2003 "Testing methods for compressive strength of fine ceramics". The test temperature is set at 23°C±5°C.

[Embodiment 2: Member for Medical Use]

A member for medical use according to one embodiment (hereinafter, also referred to as "Embodiment 2") of the present disclosure is a member for medical use, the member including the titanium material described in Embodiment 1. The member for medical use according to Embodiment 2 can have a high electrical resistivity. It is presumed that the shape of a surface of the member for medical use according to Embodiment 2 can be facilitated to be controlled by anodization treatment. Examples of the member for medical use include a dental implant component member, an artificial joint, housing and component member of a body implantable device.

[Embodiment 3: Dental Implant Component Member]

A dental implant component member according to one embodiment (hereinafter, also referred to as "Embodiment 3") of the present disclosure is a dental implant component member including the titanium material described in Embodiment 1. The dental implant component member according to Embodiment 3 can have a high electrical resistivity. It is presumed that the shape of the surface of the dental implant component member according to Embodiment 3 is facilitated to be controlled by the anodization treatment.

[Embodiment 4: Capsule for Accommodating Diamond Sensor]

A capsule for accommodating a diamond sensor according to one embodiment (hereinafter, also referred to as "Embodiment 4") of the present disclosure is a capsule for accommodating a diamond sensor, the capsule including the titanium material described in Embodiment 1. The titanium material of Embodiment 1 can have a high electrical resistivity. The titanium material can be suitably used as a material of the capsule for accommodating a diamond sensor.

[Embodiment 5: Method for Manufacturing Titanium Material]

A method (hereinafter, also referred to as "Embodiment 5") for manufacturing the titanium material described in Embodiment 1 will be described below.

<Ultrahigh-Temperature and High-Pressure Apparatus>

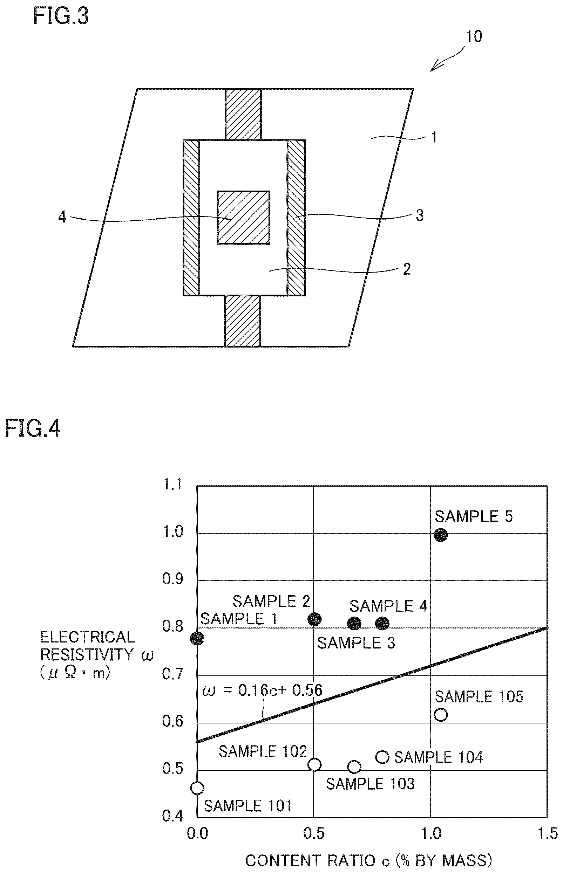

An ultrahigh-pressure and high-temperature apparatus used to manufacture the titanium material according to Embodiment 5 will be described with reference to Fig. 3. Fig. 3 is a schematic cross section of a high-pressure cell of the ultrahigh-pressure and high-temperature apparatus used in Embodiment 5. A high-pressure cell 10 includes a pressure medium 1 having a regular octahedral shape, a sample container 2 disposed inside pressure medium 1, and a heating element 3 disposed around sample container 2. Sample container 2 consists of hexagonal boron nitride. Heating element 3 consists of graphite. A raw material 4 is sealed in sample container 2. The maximum load of the ultrahigh-pressure and high-temperature apparatus used in Embodiment 5 is, for example, 2800 tons.

<<Preparation of Raw Material>>

As a raw material, a conventional alpha pure titanium including 91% by mass or more of titanium is prepared. The titanium in the alpha pure titanium is alpha titanium having a crystal structure of an alpha phase.

<<High-Pressure and High-Temperature Treatment>>

The raw material is placed in a sample container composed of a hexagonal boron nitride polycrystal, is fed with pressure to 5 GPa at a room temperature using the ultrahigh-pressure and high-temperature apparatus, and is then heated to 300°C. Thereafter, the raw material is further fed with pressure to 7 GPa to 9 GPa, is then heated to 400°C to 600°C, and is held for 10 minutes to 20 minutes. With this, the titanium material of the present disclosure is obtained.

Since the synthetic pressure is 7 GPa to 9 GPa and the maximum load of the manufacturing apparatus is 2800 tons in the manufacturing method according to Embodiment 5, a large-sized titanium material having a cylindrical shape with a diameter of 8 mm, a height of 10 mm, and a volume of 500 mm$^3$ or more can be produced, for example. Since the titanium material has a sufficient size, a test piece for measuring electrical resistivity can be produced.

It should be noted that in each of Sawahata, et al., (2018) "Synthesis of Single Phase Polycrystals of ω-Ti, ω-Zr and Evaluating its Mechanical Properties", The Review of High Pressure Science and Technology, 28, Special Issue (hereinafter, also referred to as "reference literature 1") and Sawahata, et al., (2019) "Fabrication of single-phase polycrystalline ω-Ti under High Pressure and Evaluating its Bending Properties", The Review of High Pressure Science and Technology, 29, Special Issue, 93 (hereinafter, also referred to as "reference literature 2"), it is disclosed that by using a multianvil high-pressure apparatus (maximum load: 1000 tons), ω-Ti was produced by treating a commercially available α-Ti at 12 GPa and 400°C for 3 hours. Further, in each of reference literature 1 and reference literature 2, as a heating element of the ultrahigh-pressure and high-temperature apparatus, a lanthanum chromite oxide ($LaCr_2O_3$, thermal conductivity: 5 W/(m•K) or less) is used; and as a sample container, a magnesia (MgO: 60 W/(m•K)) is used. Since each of these materials has low thermal conductivity, a temperature gradient is likely to occur around the raw material during the high-pressure and high-temperature treatment. Moreover, since each of these materials has high hardness, a pressure gradient is also likely to occur. It is presumed that the ω-Ti produced in each of reference literature 1 and reference literature 2 has a lower electrical resistivity than that of the titanium material of the present disclosure because phase transition to the ω phase is insufficient and the α phase remains.

Furthermore, the titanium material obtained in each of reference literature 1 and reference literature 2 is small (cylindrical shape with a diameter of 4 mm, a height of 3 mm, and a volume of 37.7 mm$^3$), and therefore it is impossible to produce a test piece for measuring electrical resistivity. Since the manufacturing conditions of each of reference literature 1 and reference literature 2 use the pressure of 12 GPa, it is difficult to increase the size of the titanium material.

Examples

The present embodiment will be described more specifically by way of examples. However, the present embodiment is not limited by these examples.

[Samples 1 to 8]

<Manufacture of Titanium Material>

As a raw material of each sample, alpha pure titanium having a composition described in the column "Raw Material Composition" in Table 1 was prepared. The titanium included in the raw material of each sample is alpha titanium (α-Ti). In a sample 1, the total of the raw material is 100.0001% by mass, which is due to a round up/down relation.

The raw material of each sample was placed in a sample container composed of a hexagonal boron nitride polycrystal, was fed with pressure to 5 GPa at a room temperature using a multianvil ultrahigh-pressure and high-temperature apparatus ("mavo press LPR 1000-400/50" provided by Voggenreiter; the heating element is composed of graphite; and the maximum load is 2800 tons), and was then heated to 300°C. Thereafter, the raw material was further fed with pressure to a pressure described in the column "Pressure" of Table 1, was heated to a temperature described in the column "Temperature" of Table 1, and was held for a time described in the "Holding Time" of Table 1, thereby obtaining a titanium material. The obtained titanium material had a cylindrical shape having a size with a diameter of about 8 mm, a height of about 10 mm, and a volume of about 500 mm$^3$.

[Samples 101 to 108]

Each of samples 101 to 108 corresponds to the alpha pure titanium, which is the raw material of each of samples 1 to 8. Sample 102 corresponds to JIS-1, sample 103 corresponds to JIS-2, sample 104 corresponds to JIS-3, and each of samples 105 and 107 corresponds to JIS-4. Samples 101, 106, and 108 were prepared for the present example. In sample 101, the total of the raw material is 100.0001% by mass, which is due to a round up/down relation.

[Table 1]

Table 1

| Sample No. | Raw Material Composition | | | | | | High-Temperature and High-Pressure Treatment | | |
|---|---|---|---|---|---|---|---|---|---|
| | α-Ti | Fe | H | C | N | O | Pressure | Temperature | Holding Time |
| | % by mass | % by mass | % by mass | % by mass | % by mass | % by mass | GPa | °C | minute |
| 1 | 99.9990 | 0.0005 | 0.0001 | 0.0001 | 0.0001 | 0.0003 | 8 | 500 | 15 |
| 2 | 99.4950 | 0.2000 | 0.0150 | 0.0800 | 0.0300 | 0.1800 | 8 | 500 | 15 |
| 3 | 99.3250 | 0.3000 | 0.0150 | 0.0800 | 0.0300 | 0.2500 | 8 | 500 | 15 |
| 4 | 99.2050 | 0.3000 | 0.0150 | 0.0800 | 0.0500 | 0.3500 | 8 | 500 | 15 |
| 5 | 98.9550 | 0.5000 | 0.0150 | 0.0800 | 0.0500 | 0.4000 | 8 | 500 | 15 |
| 6 | 91.0000 | 4.3100 | 0.1300 | 0.6900 | 0.4300 | 3.4400 | 8 | 500 | 15 |
| 7 | 98.9550 | 0.5000 | 0.0150 | 0.0800 | 0.0500 | 0.4000 | 7 | 400 | 10 |
| 8 | 95.0000 | 2.4000 | 0.0700 | 0.3800 | 0.2400 | 1.9100 | 8 | 500 | 15 |
| 101 | 99.9990 | 0.0005 | 0.0001 | 0.0001 | 0.0001 | 0.0003 | - | - | - |
| 102 | 99.4950 | 0.2000 | 0.0150 | 0.0800 | 0.0300 | 0.1800 | - | - | - |
| 103 | 99.3250 | 0.3000 | 0.0150 | 0.0800 | 0.0300 | 0.2500 | - | - | - |
| 104 | 99.2050 | 0.3000 | 0.0150 | 0.0800 | 0.0500 | 0.3500 | - | - | - |
| 105 | 98.9550 | 0.5000 | 0.0150 | 0.0800 | 0.0500 | 0.4000 | - | - | - |
| 106 | 91.0000 | 4.3100 | 0.1300 | 0.6900 | 0.4300 | 3.4400 | - | - | - |
| 107 | 98.9550 | 0.5000 | 0.0150 | 0.0800 | 0.0500 | 0.4000 | - | - | - |
| 108 | 95.0000 | 2.4000 | 0.0700 | 0.3800 | 0.2400 | 1.9100 | - | - | - |

<Evaluation>

For the titanium material of each sample, the content ratio of the titanium (Ti), the percentage of the omega titanium of the titanium based on mass (shown in the column "ω-Ti/Ti" in Table 2), the content ratio c of the component of the titanium material other than the titanium, the type of the impurity element, the total content ratio of the titanium and the impurity element in the titanium material (Total Content Ratio of Ti + Impurity Element), the electrical resistivity ω, the tensile strength σB, the fracture elongation δ, the average grain diameter of the crystal grains constituting the titanium material, D90/D10, the Vickers hardness, the heat-resistant temperature (only in samples 1 to 8), the volume, the 0.2% yield strength in the tensile test, and the 0.2% yield strength in the compression test were measured. A method for measuring each of the measurement items is as described in Embodiment 1. The results are shown in Tables 2 to 4.

Regarding the titanium material of each sample, it was confirmed whether or not the electrical resistivity ω (μΩ·m) of the titanium material and the content ratio c (% by mass) of the component of the titanium material other than the titanium satisfy a relation of the following formula I:

$$\omega > 0.16c + 0.56 \qquad \text{Formula I,}$$

and

in the formula I, c is 0 or more and 9 or less.

The relation between the electrical resistivity ω (g/cm$^3$) of the titanium material of each sample and the content ratio c (% by mass) of the component of the titanium material other than the titanium is shown in the coordinate system of Fig. 4. In the coordinate system of Fig. 4, the X axis represents the content ratio c (% by mass) of the component other than the titanium, and the Y axis represents the electrical resistivity $\omega$ ($\mu\Omega\cdot$m). In Fig. 4, a region indicating the relation of the formula I is a region indicated by oblique lines.

It is confirmed that the titanium material of each of samples 1 to 8 satisfies the relation of the formula I, and the alpha pure titanium of each of samples 101 to 108 does not satisfy the relation of the formula I.

It was confirmed whether or not the titanium material of each sample satisfies a relation of the following formula II:

$$\sigma B \geq 1600 - 30\delta \qquad \text{Formula II,}$$

and

in the formula II, $\sigma B \geq 400$ and $\delta \geq 20$.

In the column "Formula I" in Table 3, "Yes" indicates that the relation of the formula I is satisfied, and "No" indicates that the relation of the formula I is not satisfied. In the column "Formula II" in Table 3, "Yes" indicates that the relation of the formula II is satisfied, and "No" indicates that the relation of the formula II is not satisfied.

[Table 2]

Table 2

| Sample No. | Titanium Material Composition | | | | |
|---|---|---|---|---|---|
| | Ti | $\omega$-Ti/Ti | Content Ratio c of Component Other Than Ti | Impurity Element | Total Content Ratio of Ti + Impurity Element |
| | % by mass | % | % by mass | Type | % by mass |
| 1 | 99.999 | 100 | 0.001 | H,C,N,O,Fe | 100 |
| 2 | 99.495 | 100 | 0.505 | H,C,N,O,Fe | 100 |
| 3 | 99.325 | 100 | 0.675 | H,C,N,O,Fe | 100 |
| 4 | 99.205 | 100 | 0.795 | H,C,N,O,Fe | 100 |
| 5 | 98.955 | 100 | 1.045 | H,C,N,O,Fe | 100 |
| 6 | 91.000 | 100 | 9.000 | H,C,N,O,Fe | 100 |
| 7 | 98.955 | 50 | 1.045 | H,C,N,O,Fe | 100 |
| 8 | 95.000 | 100 | 5.000 | H,C,N,O,Fe | 100 |
| 101 | 99.999 | 0 | 0.001 | H,C,N,O,Fe | 100 |
| 102 | 99.495 | 0 | 0.505 | H,C,N,O,Fe | 100 |
| 103 | 99.325 | 0 | 0.675 | H,C,N,O,Fe | 100 |
| 104 | 99.205 | 0 | 0.795 | H,C,N,O,Fe | 100 |
| 105 | 98.955 | 0 | 1.045 | H,C,N,O,Fe | 100 |
| 106 | 91.000 | 0 | 9.000 | H,C,N,O,Fe | 100 |
| 107 | 98.955 | 0 | 1.045 | H,C,N,O,Fe | 100 |
| 108 | 95.000 | 0 | 5.000 | H,C,N,O,Fe | 100 |

[Table 3]

Table 3

| Sample No. | Titanium Material | | | | |
|---|---|---|---|---|---|
| | Electrical Resistivity ω | Formula I | Tensile Strength σB | Fracture Elongation δ | Formula II |
| | μΩ·m | Yes/No | MPa | % | Yes/No |
| 1 | 0.78 | Yes | 500 | 38 | Yes |
| 2 | 0.82 | Yes | 900 | 32 | Yes |
| 3 | 0.81 | Yes | 950 | 32 | Yes |
| 4 | 0.81 | Yes | 1000 | 31 | Yes |
| 5 | 1.00 | Yes | 1100 | 30 | Yes |
| 6 | 2.20 | Yes | 1100 | 20 | Yes |
| 7 | 0.80 | Yes | 1000 | 30 | Yes |
| 8 | 1.60 | Yes | 1100 | 25 | Yes |
| 101 | 0.46 | No | 220 | 40 | No |
| 102 | 0.51 | No | 250 | 38 | No |
| 103 | 0.51 | No | 350 | 35 | No |
| 104 | 0.53 | No | 450 | 33 | No |
| 105 | 0.62 | No | 550 | 25 | No |
| 106 | 1.60 | No | 600 | 20 | No |
| 107 | 0.60 | No | 550 | 25 | No |
| 108 | 1.10 | No | 800 | 15 | No |

[Table 4]

Table 4

| Sample No. | Titanium Material | | | | | | |
|---|---|---|---|---|---|---|---|
| | Average Grain Diameter | D90/D10 | Vickers Hardness | Heat-Resistant Temperature | Volume | Tensile Test 0.2% Yield Strength | Compression Test 0.2% Yield Strength |
| | μm | | Hv | °C | mm³ | MPa | MPa |
| 1 | 50 | 6 | 130 | 180 | 500 | 300 | 580 |
| 2 | 20 | 5 | 230 | 180 | 500 | 600 | 900 |
| 3 | 15 | 5 | 240 | 180 | 500 | 730 | 1100 |
| 4 | 10 | 5 | 260 | 180 | 500 | 870 | 1300 |
| 5 | 5 | 5 | 280 | 180 | 500 | 1000 | 1500 |
| 6 | 5 | 5 | 280 | 180 | 500 | 1000 | 1500 |
| 7 | 5 | 5 | 240 | 180 | 500 | 800 | 1100 |
| 8 | 5 | 5 | 280 | 180 | 500 | 1000 | 1500 |
| 101 | 500 | 10 | 70 | - | 500 | 130 | 200 |
| 102 | 50 | 10 | 130 | - | 500 | 200 | 370 |
| 103 | 40 | 10 | 160 | - | 500 | 300 | 570 |
| 104 | 30 | 10 | 180 | - | 500 | 400 | 900 |

(continued)

| Sample No. | Titanium Material | | | | | | |
| | Average Grain Diameter | D90/D10 | Vickers Hardness | Heat-Resistant Temperature | Volume | Tensile Test 0.2% Yield Strength | Compression Test 0.2% Yield Strength |
| | μm | | Hv | °C | mm³ | MPa | MPa |
| 105 | 20 | 10 | 200 | - | 500 | 500 | 950 |
| 106 | 20 | 10 | 200 | - | 500 | 550 | 950 |
| 107 | 20 | 10 | 200 | - | 500 | 500 | 950 |
| 108 | 20 | 10 | 200 | | 500 | 700 | 950 |

<Review>

The titanium material of each of samples 1 to 8 corresponds to an example of the present disclosure, and the alpha pure titanium of each of samples 101 to 108 corresponds to a comparative example.

Sample 1 and sample 101 have the same content ratio of the titanium. It was confirmed that sample 1 has a higher electrical resistivity than that of sample 101.

Sample 2 and sample 102 have the same content ratio of the titanium. It was confirmed that sample 2 has a higher electrical resistivity than that of sample 102.

Sample 3 and sample 103 have the same content ratio of the titanium. It was confirmed that sample 3 has a higher electrical resistivity than that of sample 103.

Sample 4 and sample 104 have the same content ratio of the titanium. It was confirmed that sample 4 has a higher electrical resistivity than that of sample 104.

Sample 5 and sample 105 have the same content ratio of the titanium. It was confirmed that sample 5 has a higher electrical resistivity than that of sample 1051.

Sample 6 and sample 106 have the same content ratio of the titanium. It was confirmed that sample 6 has a higher electrical resistivity than that of sample 106.

Sample 7 and sample 107 have the same content ratio of the titanium. It was confirmed that sample 7 has a higher electrical resistivity than that of sample 107.

Sample 8 and sample 108 have the same content ratio of the titanium. It was confirmed that sample 8 has a higher electrical resistivity than that of sample 108.

In view of the above results, it was confirmed that the strength of the titanium material of each of samples 1 to 8 is higher than the strength of the titanium material which has the same content ratio of the titanium and in which the titanium is the alpha titanium, and the titanium material of each of samples 1 to 8 has a high strength.

Although the embodiments and examples of the present disclosure have been described as above, it is also initially expected to appropriately combine or various modify the configurations of the above-described embodiments and examples.

The embodiments and examples disclosed herein are illustrative and non-restrictive in any respect. The scope of the present invention is defined by the terms of the claims, rather than the embodiments and examples described above, and is intended to include any modifications within the scope and meaning equivalent to the terms of the claims.

REFERENCE SIGNS LIST

1 pressure medium; 2 sample container; 3 heating element; 4 raw material; 10 high-pressure cell.

**Claims**

1. A titanium material comprising 91% by mass or more of titanium, wherein

an electrical resistivity $\omega\mu\Omega\cdot$m of the titanium material and a content ratio c % by mass of a component of the titanium material other than the titanium indicates a relation of the following formula I:

$$\omega > 0.16c + 0.56 \qquad \text{Formula I,}$$

and
in the formula I, c is 0 or more and 9 or less.

2. The titanium material according to claim 1, wherein the titanium material includes 49% by mass or more of titanium having a crystal structure of an omega phase.

3. The titanium material according to claim 1 or 2, wherein the titanium material includes 98.8% by mass or more of the titanium.

4. The titanium material according to any one of claims 1 to 3, wherein

a tensile strength $\sigma B$ MPa of the titanium material and a fracture elongation $\delta$ % of the titanium material indicate a relation of the following formula II:

$$\sigma B \geq 1600\text{-}30\delta \qquad \text{Formula II,}$$

and
in the formula II, $\sigma B \geq 400$ and $\delta \geq 20$.

5. The titanium material according to any one of claims 1 to 4, wherein an average grain diameter of crystal grains constituting the titanium material is 1 $\mu$m or more and 1000 $\mu$m or less.

6. The titanium material according to any one of claims 1 to 5, wherein a Vickers hardness of the titanium material is 200 Hv or more.

7. The titanium material according to any one of claims 1 to 6, wherein a heat-resistant temperature of the titanium material is 100°C or more.

8. The titanium material according to any one of claims 1 to 7, wherein a volume of the titanium material is 0.001 mm$^3$ or more.

9. The titanium material according to any one of claims 1 to 8, wherein

the titanium material includes 98.8% by mass or more of the titanium,
the titanium material includes at least one impurity element selected from a group consisting of hydrogen, carbon, nitrogen, oxygen, and iron, and
a total content ratio of the titanium and the impurity element in the titanium material is 99.99% by mass or more.

10. The titanium material according to any one of claims 1 to 9, wherein a proportion D90/D10 of a cumulative 90% grain diameter D90 from a small diameter side to a cumulative 10% grain diameter D10 from the small diameter side in a cumulative grain size distribution based on volume of crystal grains constituting the titanium material is 5 or more and 1000 or less.

11. A member for medical use, the member comprising the titanium material according to any one of claims 1 to 10.

12. A dental implant component member comprising the titanium material according to any one of claims 1 to 10.

13. A capsule for accommodating a diamond sensor, the capsule comprising the titanium material according to any one of claims 1 to 10.

FIG.1

FIG.2

FIG.3

FIG.4

## INTERNATIONAL SEARCH REPORT

| | International application No. |
|---|---|
| | **PCT/JP2023/018481** |

**A.  CLASSIFICATION OF SUBJECT MATTER**

*C22C 14/00*(2006.01)i; *C22F 1/00*(2006.01)i; *C22F 1/18*(2006.01)i; *A61K 6/84*(2020.01)i; *A61L 27/06*(2006.01)i; *A61B 5/00*(2006.01)i
FI:   C22C14/00 Z; C22F1/18 H; C22F1/00; A61K6/84; A61L27/06; A61B5/00 Q

According to International Patent Classification (IPC) or to both national classification and IPC

**B.  FIELDS SEARCHED**

Minimum documentation searched (classification system followed by classification symbols)

C22C14/00; C22F1/00; C22F1/18; A61K6/84; A61L27/06; A61B5/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C.  DOCUMENTS CONSIDERED TO BE RELEVANT**

| Category* | Citation of document, with indication, where appropriate, of the relevant passages | Relevant to claim No. |
|---|---|---|
| X | JP 2016-094663 A (TOHO TITANIUM CO., LTD.) 26 May 2016 (2016-05-26)<br>  paragraphs [0048]-[0051] | 1, 3, 8-9 |
| A | | 2, 4-7, 10-13 |
| X | JP 4-358030 A (KABUSHIKI KAISHA TOSHIBA) 11 December 1992 (1992-12-11)<br>  paragraphs [0051]-[0052] | 1, 3, 9 |
| A | | 2, 4-8, 10-13 |
| X | JP 2009-228053 A (DAIDO STEEL CO., LTD.) 08 October 2009 (2009-10-08)<br>  paragraphs [0047], [0062]-[0086], example 1 | 1-3, 6-9, 11 |
| Y | | 12-13 |
| A | | 4-5, 10 |
| Y | JP 2015-513610 A (SMITH & NEPHEW, INC.) 14 May 2015 (2015-05-14)<br>  paragraph [0057] | 12 |
| Y | US 2020/0237269 A1 (LEE, In-Han) 30 July 2020 (2020-07-30)<br>  paragraphs [0036]-[0047], fig. 2-3 | 13 |

✓ Further documents are listed in the continuation of Box C.      ✓ See patent family annex.

| | |
|---|---|
| * Special categories of cited documents: | "T"  later document published after the international filing date or priority date and not in conflict with the application but cited to understand the principle or theory underlying the invention |
| "A"  document defining the general state of the art which is not considered to be of particular relevance | |
| "E"  earlier application or patent but published on or after the international filing date | "X"  document of particular relevance; the claimed invention cannot be considered novel or cannot be considered to involve an inventive step when the document is taken alone |
| "L"  document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O"  document referring to an oral disclosure, use, exhibition or other means | |
| "P"  document published prior to the international filing date but later than the priority date claimed | "&"  document member of the same patent family |

| Date of the actual completion of the international search | Date of mailing of the international search report |
|---|---|
| **31 July 2023** | **08 August 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT

International application No.

**PCT/JP2023/018481**

### C. DOCUMENTS CONSIDERED TO BE RELEVANT

| Category* | Citation of document, with indication, where appropriate, of the relevant passages | Relevant to claim No. |
|---|---|---|
| Y | WO 2008/090925 A1 (ARKRAY, INC.) 31 July 2008 (2008-07-31)<br>paragraphs [0074]-[0075] | 13 |
| A | JP 2011-099146 A (MITSUBISHI MATERIALS CORP.) 19 May 2011 (2011-05-19) | 1-13 |
| E, X | WO 2023/100603 A1 (SUMITOMO ELECTRIC INDUSTRIES, LTD.) 08 June 2023<br>(2023-06-08)<br>paragraphs [0007]-[0031], [0121]-[0147] | 1-12 |

Form PCT/ISA/210 (second sheet) (January 2015)

## INTERNATIONAL SEARCH REPORT
### Information on patent family members

| International application No. |
| --- |
| **PCT/JP2023/018481** |

| Patent document cited in search report | | | Publication date (day/month/year) | Patent family member(s) | Publication date (day/month/year) |
| --- | --- | --- | --- | --- | --- |
| JP | 2016-094663 | A | 26 May 2016 | (Family: none) | |
| JP | 4-358030 | A | 11 December 1992 | US 5196916 A column 10, lines 13-45 EP 0442752 A1 | |
| JP | 2009-228053 | A | 08 October 2009 | (Family: none) | |
| JP | 2015-513610 | A | 14 May 2015 | WO 2013/126407 A1 paragraph [0057] US 2015/0030493 A1 CN 104105510 A | |
| US | 2020/0237269 | A1 | 30 July 2020 | WO 2017/082573 A1 paragraphs [0035]-[0046], fig. 2-3 KR 10-2017-0054880 A | |
| WO | 2008/090925 | A1 | 31 July 2008 | US 2011/0036729 A1 paragraphs [0107]-[0108] EP 2116844 A1 CN 101809437 A KR 10-2010-0041697 A | |
| JP | 2011-099146 | A | 19 May 2011 | (Family: none) | |
| WO | 2023/100603 | A1 | 08 June 2023 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009228053 A

**Non-patent literature cited in the description**

- **HIDEKI FUJII ; TAKASHI MAEDA**. Titanium Alloys Developed by Nippon Steel & Sumitomo Metal Corporation. *Shinnittetsu Sumikin giho*, 2013, vol. 396, 16-22
- **SAWAHATA et al.** Synthesis of Single Phase Polycrystals of ω-Ti, ω-Zr and Evaluating its Mechanical Properties. *The Review of High Pressure Science and Technology*, 2018, vol. 28
- **SAWAHATA et al.** Fabrication of single-phase polycrystalline ω-Ti under High Pressure and Evaluating its Bending Properties. *The Review of High Pressure Science and Technology*, 2019, vol. 29 (93)